# EUROPEAN PATENT APPLICATION

(11) **EP 2 596 789 A1**
(43) Date of publication of application: **29.05.2013**
(21) Application number: 11809758.3
(22) Date of filing: 22.07.2011
(51) Int. Cl.: A61K 31/4415, A61P 1/00, A61P 7/00, A61P 7/08, C07D 213/66

(54) **ORAL MEDICINAL COMPOSITION FOR PATIENTS UNDERGOING PERITONEAL DIALYSIS AND METHOD FOR USING SAME**

(30) Priority: 23.07.2010 JP 2010166148
(71) Applicant: Tokai University Educational System, Tokyo 151-0063 (JP)
(72) Inventor: KAKUTA, Takatoshi, Isehara-shi Kanagawa 259-1193 (JP); MIYATA, Toshio, Isehara-shi Kanagawa 259-1193 (JP)
(74) Representative: Vossius & Partner
(86) International application number: PCT/JP2011/066763
(87) International publication number: WO 2012/011588

(57) **Abstract**

The present invention provides an oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid, the oral medicinal composition comprising a pharmaceutically acceptable salt of pyridoxamine as an active ingredient.

## Description

### Technical Field

The present invention relates to a medicinal composition that is orally administered to patients who undergo peritoneal dialysis (peritoneal dialysis patients), such as chronic renal failure patients, and a method for using the medicinal composition. More preferably, the present invention relates to a medicinal composition that is used to protect the peritoneal tissue of peritoneal dialysis patients from carbonyl stress caused by peritoneal dialysis to thereby suppress or improve peritoneal dysfunction or ultrafiltration failure, and a method for using the medicinal composition.

### Background Art

Peritoneal dialysis for renal failure has advantages, such as ease of use and relatively shorter unfree time; however, it is known that continuous peritoneal dialysis leads to a gradual decline in water-removing function, causing denaturation and curing of peritoneal proteins, and peritoneal fusion.

Since peritoneal dialysis uses the patient's own peritoneum as a dialysis membrane, carbonyl compounds present in the peritoneal dialysis fluid injected into the peritoneal cavity (exogenous carbonyl compounds) and carbonyl compounds present in the blood traveling from the body (endogenous carbonyl compounds) create an environment of carbonyl stress in the peritoneal cavity and peritoneum tissue. Therefore, the peritoneum is always exposed to carbonyl compounds, which are uremic toxins. In peritoneal dialysis therapy continued for a long period of time, peritoneal disorders quite often cause a decline in peritoneal function. If the disorders continue, peritoneal dysfunction occurs; in a worst-case scenario, severe peritonitis occurs, sometimes resulting in death.

Long-term peritoneal dialysis patients, such as chronic renal failure patients, develop a gradual decline in peritoneal function with characteristic symptoms of an accelerated loss of glucose-dependent osmotic pressure gradient across the peritoneum, and a loss of ultrafiltration ability (NPL 1 and NPL 2). The decreased peritoneal function increases the functional area (so-called "effective peritoneal surface area (EPSA)") of the exchange of small-molecule solutes (urea, creatinine, and glucose) between the blood and dialysis fluid (NPL 3). This change is attributable to enhanced angiogenesis, and presumably to peritoneal vasodilatation (NPL 4).

Since many conventionally used peritoneal dialysis fluids contain glucose as an osmoregulator, the above phenomena are considered to be partially attributable to the glucose contained in the peritoneal dialysis fluids. Glucose is unstable in heat. Part of the glucose is degraded during sterilization in the process of manufacture of peritoneal dialysis fluids, and very highly reactive carbonyl compounds that can easily modify proteins are accumulated as degradation products. Moreover, peritoneal dialysis fluids containing glucose are considered to accumulate reactive carbonyl compounds as degradation products during storage after sterilization.

Generally, degradation of glucose is likely to occur in the neutral to alkaline range. Thus, in consideration of the stability of glucose, a buffer system that provides a pH in the acidic range (pH 5 to 5.4) is often used in common peritoneal dialysis fluids. However, such acid peritoneal dialysis fluids raise concerns about reduction in the immunological defense mechanism of peritoneal macrophages, development of peritonitis caused by the entrance of bacteria, disorders of peritoneal mesothelial cells, etc. In order to solve such conflicting problems, there have been demands for fundamentally suppressing the production of carbonyl compounds due to glucose degradation in peritoneal dialysis fluids, or removing carbonyl compounds from peritoneal dialysis fluids.

Many studies have been performed so far to solve these problems. For example, a two-compartment mixing type dialysis fluid has been developed (PTL 1). More specifically, a dialysis fluid bag is separated into two compartments, and a partition structure that can open at the point of use is provided between the two compartments. One compartment is filled with a glucose solution adjusted to an acidic pH (pH 3.5 to 4.5), at which glucose is difficult to degrade by heating for sterilization, and the other compartment is filled with an electrolyte solution having a pH of 6.5 to 7.7. When the dialysis fluid bag is used, the partition is opened to mix the glucose solution and the electrolyte so that the mixture is used as a dialysis fluid with a pH of 6.5 to 7.7.

Further, for the reason that a peritoneal dialysis fluid containing high-concentration glucose is not suitable for the peritoneum because, for example, carbonyl compounds produced by the degradation of glucose modify proteins, a peritoneal dialysis fluid using a glucose polymer, which produces less amounts of decomposition products, is developed (PTL 2). Moreover, from the same viewpoint, a dialysis fluid using cyclodextrin in place of glucose as an osmoregulator (PTL 3), a peritoneal dialysis fluid using a disaccharide (PTL 4), and a peritoneal dialysis fluid using an amino acid (NPL 5) have also been proposed. In addition, a peritoneal dialysis fluid to which cysteine is added to suppress the degradation of glucose (PTL 5) has also been disclosed.

As still another method, a method of previously adding, to a peritoneal dialysis fluid, a drug for eliminating carbonyl compounds produced in the production process of the dialysis fluid has also been developed. For example, peritoneal dialysis fluids to which aminoguanidine, pyridoxamine, a biguanide compound, or an SH compound is previously added to improve the carbonyl stress state in the peritoneal dialysis fluids have been proposed (PTL 6, PTL 7, and PTL 8).

All of these techniques are intended to previously add pyridoxamine or other carbonyl-eliminating agents to peritoneal dialysis fluids to thereby previously eliminate carbonyl compounds produced in the production process of the peritoneal dialysis fluids. That is, these techniques are designed to eliminate and minimize carbonyl compounds in peritoneal dialysis fluids, thereby decreasing the influence of carbonyl compounds on the peritoneum to protect the peritoneum.

The molecular mechanism that is the basis of peritoneal dysfunction has not yet been clarified. Relapse of peritonitis accompanied by inflammatory changes causes damage to the peritoneum over a long period of time (NPL 1 and NPL 5); however, the relapse is not a condition for the development of ultrafiltration failure (NPL 4 and NPL 6). Recent studies have been clarifying the mechanism of the decline in peritoneal function of long-term peritoneal dialysis patients (NPL 7 to NPL 14). Chronic uremic toxins themselves change the peritoneum and enlarge the effective peritoneal surface area (NPL 8). Biochemical changes in the peritoneum are considered to be at least partly caused by the overload of very highly reactive carbonyl compounds (peritoneal carbonyl stress) due to both uremic toxin circulation and the peritoneal dialysis fluid (NPL 9 to NPL 14). Plasma carbonyl compounds are accumulated during the circulation of uremic toxins, and gradually dispersed in the peritoneal cavity to initiate the modification of advanced glycation/lipoxidation end products (NPL 15 to NPL 21). During peritoneal dialysis, carbonyl compounds produced by heat sterilization of the glucose peritoneal dialysis fluid enter into the peritoneum. These are supplemented by the increased transfer of serum carbonyl compounds by the dialysis treatment itself. The carbonyl compounds are considered to further stimulate the production of cytokines and growth factors (e.g., VEGF), and to regulate the expression of nitric oxide-forming enzymes (NOS) in the peritoneal cells (NPL 22 to NPL 25). VEGF and nitric oxide (NO) cooperate to stimulate angiogenesis, increase permeability, and dilate peritoneal vessels (NPL 23 to NPL 25). These complex changes presumably enlarge the effective peritoneal surface area, so that osmotic pressure is lost earlier than normal, and finally ultrafiltration is not performed. Upregulation of FGF-2 and TGF-β also stimulate peritoneal interstitial fibrosis (NPL 24 to NPL 26).

### Citation List

### Patent Literature

PTL 1: WO 93/09820
PTL 2: Japanese Unexamined Patent Publication No. 10-94598
PTL 3: Japanese Unexamined Patent Publication No. 8-71136
PTL 4: Japanese Unexamined Patent Publication No. 8-131541
PTL 5: Japanese Unexamined Patent Publication No. 5-105633
PTL 6: WO 2000/10606
PTL 7: US 2003/0017995 A1
PTL 8: WO 2005/126523

### Non-patent Literature

NPL 1: Davies SJ, et al., Kidney Int., 54, 2207-2217, 1998
NPL 2: Krediet RT, et al., Perit Dial Bull 6: 61-65, 1986
NPL 3: Rippr B, et al., Peritoneal physiology: Transport of solutions. In: The Textbook of Peritoneal Dialysis, edited by Gokal R, Nolf KD, Dordrecht, The Netherlands, Kluwer Academic Publishers, 1994, 69-113
NPL 4: Krediet RT, et al., Kidney Int 55: 341-356, 1999
NPL 5: Krediet RT, et al., Ave Ren Replace Ther 5: 212-217, 1994
NPL 6: Struijk SG, et al., Kidney Int 45: 1739-1744, 1994
NPL 7: Davis SJ, et al., Nephrol Dial Transplant 11: 448-506, 1996
NPL 8: Combet S, et al., J Am Soc Nephrol 12: 2146-2157, 2001
NPL 9: Korbet SM, et al., Am J Kidney Dis 22: 588-591, 1993
NPL 10: Miyata T, et al., Kidney Int 2002; 61: 375-386
NPL 11: Nakayama M, et al., Kidney Int 51: 182-186, 1997
NPL 12: Miyata T, et al., Kidney Int 58: 425-435, 2000
NPL 13: Witowski J, et al., J Am Soc Nephrol 11: 729-739, 2000
NPL 14: Wieslander A, et al., Adv Perit Dial 12: 57-60, 1996
NPL 15: Garcia-Lopez E, et al., Perit Dial Int 20 (Suppl 5): S48-S56, 2000
NPL 16: Krediet RT, et al., Perit Dial Int 17: 35-41, 1997
NPL 17: Faller B, K, Kidney Int (Suppl 56): S81-S85, 1996
NPL 18: Rippe B, et al., Kidney Int 59: 348-357, 2001
NPL 19: Topley N, Perit Dial Int: 17: 42-47, 1997
NPL 20: Feriani M, et al., Kidney Int 54: 1731-1738, 1998
NPL 21: Lage C, et al., Perit Dial Int 20 (Suppl 5): S28-S32, 2000
NPL 22: Papapetropoulos A, et al., J Clin Invest 100: 3131-3139, 1997
NPL 23: Vriese AS, et al., J Am Soc Nephrol 12: 2029-2039, 2001
NPL 24: Margetts PJ, et al., J Am Soc Nephrol 12: 2029-39, 2001
NPL 25: Combet S, et al., J Am Soc Nephrol 11: 717-728, 2000
NPL 26: Ogata S, et al., J Am Soc Nephrol 12: 2787-2796, 2001

### Summary of Invention

### Technical Problem

Peritoneal disorders resulting from the carbonyl stress state during peritoneal dialysis are caused by various factors, as mentioned above. Peritoneal dialysis treatment causes not only carbonyl compounds in the peritoneal dialysis fluid to flow into the peritoneal cavity, but also carbonyl compounds present in the blood to be transferred into the peritoneal cavity while moving three-dimensionally and vertically through the peritoneal tissue. Nevertheless, existing techniques for improving the carbonyl stress state are merely intended to improve peritoneal dialysis fluids to thereby reduce damage to the peritoneum caused by the peritoneal dialysis fluids.

Thus, there is now a need to establish a method for reducing damage to the peritoneum by comprehensively improving the carbonyl stress state resulting from not only carbonyl compounds in the peritoneal dialysis fluid flowing into the peritoneal cavity, but also carbonyl compounds present in the blood being transferred into the peritoneal cavity while moving three-dimensionally and vertically through the peritoneal tissue due to the difference in the osmotic pressure caused by peritoneal dialysis treatment.

Furthermore, as a conventional technique for improving the carbonyl stress state caused by peritoneal dialysis fluids, a technique of previously adding pyridoxamine to a peritoneal dialysis fluid is proposed (PTL 8). However, after the present inventors conducted an experiment, it was revealed that pyridoxamine was very unstable in the peritoneal dialysis fluid, regardless of the pH of the dialysis fluid (Experimental Example 1). More specifically, using a peritoneal dialysis fluid filled in a two-compartment bag having two compartments separated by a partition, a pyridoxamine salt was dissolved in a solution in the upper compartment (acid glucose-containing solution), a solution in the lower compartment (alkaline electrolyte solution), and a neutral solution obtained by mixing these solutions (final peritoneal dialysis fluid), and their stability was examined at room temperature over three weeks. The results revealed that the stability of pyridoxamine was unexpectedly so low that the pyridoxamine contents of the upper-compartment and lower-compartment solutions, before they were mixed, were reduced to 70% of the original contents within only three weeks. Accordingly, in order to actually commercialize peritoneal dialysis fluids containing pyridoxamine, it is necessary to consider a new approach for stabilizing pyridoxamine in dialysis fluids.

In light of the above, an object of the present invention is to provide a technique for extensively improving exogenous and endogenous carbonyl stress states caused by peritoneal dialysis by another approach rather than improving peritoneal dialysis fluids, and for effectively suppressing functional decline in the peritoneum and its neighboring tissue caused by peritoneal dialysis.

### Solution to Problem

The present inventors conducted extensive research to achieve the above object, and found that when pyridoxamine in the form of a salt was orally administered to uremic rats with kidney subtotal excision as a model of patients in need of peritoneal dialysis due to chronic renal failure (peritoneal dialysis patients), and a peritoneal dialysis fluid was intraperitoneally administered (peritoneally dialyzed) to the rats, the pyridoxamine reached, with stable activity, the peritoneal cavity from the blood through the peritoneal tissue, so that the pyridoxamine was efficiently and stably transferred into the peritoneal cavity. Further, the amount of carbonyl compounds in the peritoneal cavity and the amount of advanced glycation/lipoxidation end products (AGEs) in the peritoneal tissue were significantly reduced in these uremic rats, as compared with uremic rats to which a peritoneal dialysis fluid had been intraperitoneally administered without oral administration of a salt of pyridoxamine. This confirmed that the carbonyl stress state caused by peritoneal dialysis was significantly improved. Based on these findings, the present inventors were convinced that oral administration of pyridoxamine in the form of a salt to a patient who receives peritoneal dialysis treatment can improve not only carbonyl stress states in the peritoneal cavity and peritoneal tissue caused by the flow of carbonyl compounds contained in the peritoneal dialysis fluid (exogenous carbonyl compounds) into the peritoneal cavity due to peritoneal dialysis, but also carbonyl stress states in the peritoneal cavity and peritoneal tissue caused by the flow of carbonyl compounds present in the body (uremic components circulating in the body) into the peritoneal cavity while moving through the peritoneal tissue due to peritoneal dialysis; and that consequently, functional decline in the peritoneum and its neighboring tissue caused by peritoneal dialysis can be extensively and effectively suppressed.

It has not been known that when peritoneal dialysis is performed after pyridoxamine in the form of a salt is orally administered to a peritoneal dialysis patient, the pyridoxamine can be efficiently and stably transferred into the peritoneal cavity. Moreover, it has not been investigated that when pyridoxamine in the form of a salt is orally administered, carbonyl stress states in the peritoneal tissue and peritoneal cavity can be improved.

The present invention has been accomplished on the basis of these findings, and includes the following embodiments.

### (I) Oral Medicinal Composition for Peritoneal Dialysis Patients

(I-1) An oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid, the oral medicinal composition comprising a pharmaceutically acceptable salt of pyridoxamine as an active ingredient.

(I-2) The oral medicinal composition according to (I-1), wherein the carbonyl compound is 3-deoxyglucosone.

(I-3) The oral medicinal composition according to (I-1) or (I-2), wherein the advanced glycation/lipoxidation end product (AGE) is pentosidine.

(I-4) The oral medicinal composition according to any one of (I-1) to (I-3), wherein the pharmaceutically acceptable salt of pyridoxamine is pyridoxamine dihydrochloride.

(I-5) The oral medicinal composition according to any one of (I-1) to (I-4), which is a medicinal composition for improving a state of carbonyl stress in the peritoneal cavity and peritoneal tissue.

(I-6) The oral medicinal composition according to any one of (I-1) to (I-4), which is a medicinal composition for protecting the peritoneum of a peritoneal dialysis patient.

(I-7) The oral medicinal composition according to any one of (I-1) to (I-6), which is for suppressing or/and improving peritoneal dysfunction or ultrafiltration failure in peritoneal dialysis patients.

(I-8) The oral medicinal composition according to any one of (I-1) to (I-7), wherein the glucose-containing peritoneal dialysis fluid is free from pyridoxamine or a pharmaceutically acceptable salt thereof.

### (II) Method for Suppressing Increase of Carbonyl Compounds and/or Advanced Glycation/Lipoxidation End Products (AGEs) in Peritoneal Cavity and Peritoneal Tissue of Peritoneal Dialysis Patient

(II-1) A method for suppressing an increase of carbonyl compounds and/or advanced glycation/lipoxidation end products (AGEs) in the peritoneal cavity and peritoneal tissue of a peritoneal dialysis patient (chronic renal failure patient) after intraperitoneal administration of a glucose-containing peritoneal dialysis fluid, the method comprising the steps of:
   (a) orally administering a pharmaceutically acceptable salt of pyridoxamine to the peritoneal dialysis patient;
   (b) intraperitoneally administering a glucose-containing peritoneal dialysis fluid to the patient after and/or before step (a); and
   (c) transferring the pyridoxamine or the pharmaceutically acceptable salt thereof into the peritoneal cavity.

(II-2) The method according to (II-1), wherein the carbonyl compound is 3-deoxyglucosone.

(II-3) The method according to (II-1) or (II-2), wherein the advanced glycation/lipoxidation end product (AGE) is pentosidine.

(II-4) The method according to any one of (II-1) to (II-3), wherein the pharmaceutically acceptable salt of pyridoxamine is pyridoxamine dihydrochloride.

(II-5) The method according to any one of (II-1) to (II-4), wherein the glucose-containing peritoneal dialysis fluid is free from pyridoxamine or a pharmaceutically acceptable salt thereof.

### (III) Method for Protecting Peritoneal Tissue Of Peritoneal Dialysis Patient from Carbonyl Stress Caused by Glucose-Containing Peritoneal Dialysis

(III-1) A method for protecting the peritoneal tissue of a peritoneal dialysis patient from carbonyl stress caused by glucose-containing peritoneal dialysis, the method comprising the steps of:
   (a) orally administering a pharmaceutically acceptable salt of pyridoxamine to the peritoneal dialysis patient;
   (b) intraperitoneally administering a glucose-containing peritoneal dialysis fluid to the patient after and/or before step (a);
   (c) transferring the pyridoxamine or the pharmaceutically acceptable salt thereof into the peritoneal cavity; and
   (d) suppressing an increase of carbonyl compounds and/or advanced glycation/lipoxidation end products (AGEs) in the peritoneal cavity and peritoneal tissue caused by the intraperitoneal administration of the glucose-containing peritoneal dialysis fluid.

(III-2) The method according to (III-1), wherein the carbonyl compound is 3-deoxyglucosone.

(III-3) The method according to (III-1) or (III-2), wherein the advanced glycation/lipoxidation end product (AGE) is pentosidine.

(III-4) The method according to any one of (III-1) to (III-3), wherein the pharmaceutically acceptable salt of pyridoxamine is pyridoxamine dihydrochloride.

(III-5) The method according to any one of (III-1) to (III-4), which is for suppressing or/and improving peritoneal dysfunction or ultrafiltration failure in peritoneal dialysis patients.

(III-6) The method according to any one of (III-1) to (III-5), wherein the glucose-containing peritoneal dialysis fluid is free from pyridoxamine or a pharmaceutically acceptable salt thereof.

### (IV) Pharmaceutically Acceptable Salt of Pyridoxamine Having Oral Dosage Form

(IV-1) A pharmaceutically acceptable salt of pyridoxamine having an oral dosage form for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

(IV-2) The pharmaceutically acceptable salt of pyridoxamine according to (IV-1), wherein the carbonyl compound is 3-deoxyglucosone.

(IV-3) The pharmaceutically acceptable salt of pyridoxamine according to (IV-1) or (IV-2), wherein the advanced glycation/lipoxidation end product (AGE) is pentosidine.

(IV-4) The pharmaceutically acceptable salt of pyridoxamine according to any one of (IV-1) to (IV-3), which is for improving a state of carbonyl stress in the peritoneal cavity and peritoneal tissue of a peritoneal dialysis patient.

(IV-5) The pharmaceutically acceptable salt of pyridoxamine according to any one of (IV-1) to (IV-4), which is for protecting the peritoneum of a peritoneal dialysis patient.

(IV-6) The pharmaceutically acceptable salt of pyridoxamine according to any one of (IV-1) to (IV-5), which is for suppressing or/and improving peritoneal dysfunction or ultrafiltration failure in peritoneal dialysis patients.

(IV-7) The pharmaceutically acceptable salt of pyridoxamine according to any one of (IV-1) to (IV-6), wherein the pharmaceutically acceptable salt of pyridoxamine is pyridoxamine dihydrochloride.

(IV-8) The pharmaceutically acceptable salt of pyridoxamine according to any one of (IV-1) to (IV-7), wherein the glucose-containing peritoneal dialysis fluid is free from pyridoxamine or a pharmaceutically acceptable salt thereof.

### (V) Use of Pharmaceutically Acceptable Salt of Pyridoxamine

(V-1) Use of a pharmaceutically acceptable salt of pyridoxamine for producing an oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

(V-2) The use according to (V-1), wherein the carbonyl compound is 3-deoxyglucosone.

(V-3) The use according to (V-1) or (V-2), wherein the advanced glycation/lipoxidation end product (AGE) is pentosidine.

(V-4) The use according to any one of (V-1) to (V-3), wherein the pharmaceutically acceptable salt of pyridoxamine is pyridoxamine dihydrochloride.

(V-5) The use according to any one of (V-1) to (V-4), wherein the oral medicinal composition is for improving a state of carbonyl stress in the peritoneal cavity and peritoneal tissue.

(V-6) The use according to any one of (V-1) to (V-4), wherein the oral medicinal composition is for protecting the peritoneum of a peritoneal dialysis patient.

(V-7) The use according to any one of (V-1) to (V-5), wherein the oral medicinal composition is for suppressing or/and improving peritoneal dysfunction or ultrafiltration failure in peritoneal dialysis patients.

(V-8) The use according to any one of (V-1) to (V-7), wherein the glucose-containing peritoneal dialysis fluid is free from pyridoxamine or a pharmaceutically acceptable salt thereof.

### Advantageous Effects of Invention

The peritoneum of uremic patients is characterized by increased functional area for exchanging small-molecule solutes between the blood and dialysis fluid, vascular proliferation, increased advanced glycation/lipoxidation end products, and upregulation of the expression of angiogenic cytokines (VEGF and FGF-2). Further, the peritoneal carbonyl stress caused by not only the uremic environment but the peritoneal dialysis treatment as well contributes to vascular proliferation induced by bioactive molecules, and to increased functional exchange area of small-molecule solutes, finally leading to ultrafiltration failure. This phenomenon is severer than chronic uremic patients (chronic renal failure patients) who do not undergo long-term peritoneal dialysis.

In the present invention, the pyridoxamine in the form of a salt orally administered to a uremic patient (renal failure patient) who undergoes peritoneal dialysis is stably and efficiently transferred into the blood and peritoneal cavity, and eliminates and reduces carbonyl compounds. As a result, the transport rate of small-molecule solutes and vessel density are significantly reduced, leading to suppression or improvement of peritoneal dysfunction, particularly ultrafiltration failure. This improvement of peritoneal function is accompanied by the enhancement of angiogenesis induced by uremia and peritoneal dialysis, and the suppression of the angiogenic cytokine expression. Actually, when a pyridoxamine salt is orally administered, the pentosidine content in the peritoneal tissue significantly decreases. Therefore, the orally administered pyridoxamine in the form of a salt stably and efficiently reaches the peritoneal cavity from the blood via the peritoneal tissue and improves the peritoneal function of the peritoneal dialysis patients, thereby protecting the peritoneum of the uremic patients who undergo peritoneal dialysis.

Further, the pharmaceutically acceptable salt of pyridoxamine used as an active ingredient in the present invention is a safe substance with few side effects. In the present invention, this salt is orally administered to chronic renal failure patients who undergo peritoneal dialysis, thereby effectively improving carbonyl stress states in the peritoneal cavity and peritoneal tissue caused by the peritoneal dialysis, as described above. Moreover, because the carbonyl stress states are effectively improved, functional decline in the peritoneum and its neighboring tissue caused by the peritoneal dialysis can be effectively suppressed.

Furthermore, since the medicinal composition of the present invention has an oral dosage form, such as tablets and capsules, it can be carried in a stable state and taken not only during dialysis, but at any time.

### Description of Embodiments

### (I) Oral Medicinal Composition for Peritoneal Dialysis Patients

The present invention relates to an oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

Peritoneal dialysis patients targeted by the present invention are those who are in need of peritoneal dialysis, such as chronic renal failure patients, who would develop uremia without dialysis. Moreover, the glucose-containing peritoneal dialysis fluid is a peritoneal dialysis fluid containing glucose that is used in peritoneal dialysis performed on the above-mentioned chronic renal failure patients and uremic patients, and the composition of the dialysis fluid is known in the art. Such a glucose-containing peritoneal dialysis fluid is globally commercially available. Examples thereof include "Dianeal (registered trademark) PD-2" series, "Dianeal (registered trademark) PD-4" series, "Dianeal (registered trademark)-N PD-2" series, and "Dianeal (registered trademark)-N PD-4" series, all of which are supplied from Baxter Limited, and are used in the Experimental Examples. However, the peritoneal dialysis fluid is not limited to these examples, and glucose-containing peritoneal dialysis fluids approved by pharmaceutical authorities of any country (e.g., the Ministry of Health, Labour and Welfare of Japan) can be widely used.

The above-mentioned carbonyl compounds are reactive carbonyl compounds that are produced from sugar and lipid, and non-enzymatically reacted with proteins in the body to produce advanced glycation/lipoxidation end products (AGEs). In the present invention, at least 3-deoxyglucosone is exemplified. Examples of other carbonyl compounds include glyoxal, methylglyoxal, etc.

The above-mentioned advanced glycation/lipoxidation end products (AGEs) are produced by the non-enzymatic reaction of carbonyl compounds with proteins in the body, as described above, and cause carbonyl stress. An example of an AGE is pentosidine. The substances carboxymethyllysine and pyrraline are also known as AGEs.

The medicinal composition targeted by the present invention is a medicinal composition that is applied to the above-mentioned peritoneal dialysis patients before and/or after intraperitoneal administration (peritoneal dialysis) of a glucose-containing peritoneal dialysis fluid. The medicinal composition is mainly used to suppress an increase of carbonyl compounds and/or advanced glycation/lipoxidation end products (AGEs) in the peritoneal cavity and peritoneal tissue after peritoneal dialysis.

The active ingredient used in the medicinal composition of the present invention is a salt of pyridoxamine.

The salt of pyridoxamine may be any pharmaceutically acceptable salt. Examples thereof include inorganic acid salts of pyridoxamine, such as hydrochloride, sulfate, nitrate, and phosphate; and organic acid salts of pyridoxamine, such as maleate, fumarate, tartrate, citrate, and acetate. In particular, pyridoxamine dihydrochloride is more preferable as the pyridoxamine salt used in the pharmaceutical preparation as an oral drug.

Moreover, the pyridoxamine salt can be used as a hydrate or solvate. The pyridoxamine salt can be synthesized by a conventionally known method, or can be purchased as a commercial product.

The dosage form of the medicinal composition of the present invention may be any dosage form for oral administration. Examples thereof include solid formulations, semi-solid formulations, and liquid formulations. In consideration of the stability of the pyridoxamine salt, preferred examples are solid formulations and semi-solid formulations; and more preferred examples are solid formulations. Specific examples thereof include tablets (including uncoated tablets, sugar-coated tablets, fast-disintegrating oral tablets, fast-dissolving oral tablets, chewable tablets, effervescent tablets, troches, drops, film-coated tablets, etc.), pills, granules, fine granules, powders, hard capsules, and soft capsules. Preferred examples are tablets.

The medicinal composition of the present invention may be in the form of fast-disintegrating oral tablets, fast-dissolving oral tablets, or chewable tablets, which can be easily taken without water. Alternatively, the medicinal composition of the present invention may be in the form of masticatories, sublingual formulations, buccals, troches, ointments, patches, or solutions, which can be locally administered in the oral cavity. In addition, the medicinal composition of the present invention may be modified to have sustained-release properties, stability, readily disintegrable properties, hardly disintegrable properties, enteric properties, easily absorbed properties, etc.

The medicinal composition of the present invention, which contains a salt of pyridoxamine as an active ingredient, may generally further contain carriers and additives for forming it into an oral dosage form. Moreover, in addition to the above-described active ingredient, the medicinal composition of the present invention may appropriately contain any components that can usually be used in drugs, quasi-drugs, food or drink, depending on, for example, the application and the dosage form, as long as the effect of the present invention, pharmaceutical stability, and the like are not impaired. It is preferable to select these components so that side effects of the pyridoxamine salt as an active ingredient are minimized, and drug effects of the pyridoxamine salt are not inhibited or are least affected. The preparation of the medicinal composition comprising the pyridoxamine salt in combination with a pharmaceutically acceptable carrier and/or additive can be carried out by a conventionally known method.

Examples of components that can be mixed include the following carriers and additives.

Examples of carrier components or additives that can be contained in solid formulations include excipients, disintegrators, binders, lubricants, antioxidants, coating agents, coloring agents, taste-masking agents, surfactants, plasticizers, sweetening agents, flavoring agents, disintegration aids, foaming agents, adsorbents, preservatives, wetting agents, and antistatic agents. Examples of carrier components or additives that can be contained in liquid formulations include solvents, pH adjusters, refreshing agents, suspending agents, antifoaming agents, thickening agents, and solubilizing agents; the above-mentioned surfactants, antioxidants, coloring agents, sweetening agents, and flavoring agents; and antiseptic and antibacterial agents, chelating agents, solubilizers or solubilizing agents, stabilizers, fluidizers, emulsifiers, thickeners, buffers, isotonizing agents, and dispersants.

Specific examples of components that can be used as such carriers and additives are shown below, but are not limited thereto.

Examples of excipients include sugar alcohols, such as D-sorbitol, mannitol, and xylitol; saccharides, such as glucose, sucrose, lactose, and fructose; crystalline cellulose, carmellose sodium, croscarmellose sodium, calcium hydrogen phosphate, wheat starch, rice starch, corn starch, potato starch, dextrin, β-cyclodextrin, light anhydrous silicic acid, titanium oxide, magnesium aluminometasilicate, talc, and kaolin.

Examples of disintegrators include low-substituted hydroxypropyl cellulose, carboxymethyl cellulose calcium, croscarmellose sodium, hydroxypropyl starch, and partially pregelatinized starch.

Examples of binders include methyl cellulose, ethyl cellulose, hydroxypropyl cellulose, hydroxypropyl methylcellulose, and other cellulose derivatives; polyvinyl pyrrolidone, polyvinyl alcohol, acrylic acid-based polymers, gelatin, gum arabic, pullulan, pregelatinized starch, agar, tragacanth, sodium alginate, and propylene glycol alginate.

Examples of lubricants include stearic acid, magnesium stearate, calcium stearate, polyoxyl stearate, cetanol, talc, hardened oil, sucrose fatty acid ester, dimethylpolysiloxane, beeswax, and white beeswax.

Examples of antioxidants include dibutylhydroxytoluene (BHT), propyl gallate, butylhydroxyanisole (BHA), tocopherol, and citric acid.

Examples of coating agents include hydroxypropyl methyl cellulose, hydroxypropyl cellulose, methyl cellulose, ethyl cellulose, hydroxypropyl methyl cellulose phthalate, hydroxypropyl methyl cellulose acetate succinate, carboxymethyl ethyl cellulose, cellulose acetate phthalate, polyvinyl acetal diethylaminoacetate, aminoalkyl methacrylate copolymers, hydroxypropyl methyl cellulose acetate succinate, methacrylic acid copolymers, polyvinyl acetate diethylaminoacetate, and shellac.

Examples of coloring agents include Food Red No. 2, Food Red No. 3, Food Red No. 102, Food Yellow No. 4, Food Yellow No. 5, Food Blue No. 1, Food Yellow No. 4 metal lake, sodium copper chlorophyllin, riboflavin, turmeric extract, and carotene liquid.

Examples of taste-masking agents include aspartame, ascorbic acid, stevia, menthol, crude glycyrrhiza extract, and simple syrup.

Examples of surfactants include polyoxyethylene hydrogenated castor oil, glyceryl monostearate, sorbitan monostearate, sorbitan monolaurate, polyoxyethylene polyoxypropylene, polysorbates, sodium lauryl sulfate, macrogols, and sucrose fatty acid ester.

Examples of plasticizers include triethyl citrate, polyethylene glycol, triacetin, and cetanol.

Examples of sweetening agents include natural or synthetic sweetening agents, such as sucrose, mannitol, and aspartame.

Examples of flavoring agents include camphor, borneol, and cinnamaldehyde.

Examples of solvents include water, ethanol, isopropanol, lauryl alcohol, cetanol, stearyl alcohol, oleyl alcohol, lanolin alcohol, behenyl alcohol, 2-hexyldecanol, isostearyl alcohol, and 2-octyldodecanol.

Examples of pH adjusters include citric acid, malic acid, sodium hydrogen phosphate, and dipotassium phosphate.

Examples of suspending agents include kaolin, carmellose sodium, xanthan gum, methyl cellulose, and tragacanth.

Examples of antifoaming agents include dimethylpolysiloxane and silicone antifoaming agents.

Examples of thickening agents include xanthan gum, tragacanth, methylcellulose, and dextrin.

Examples of solubilizing agents include ethanol, sucrose fatty acid ester, and macrogol.

The production method of the medicinal composition of the present invention is not limited, and the medicinal composition of the present invention can be produced by a method commonly used in the art, without modification or with appropriate modification.

For example, tablets can be prepared by mixing a powdered active ingredient and a pharmaceutically acceptable carrier component (e.g., an excipient), and directly compression-molding the mixture (direct tableting method). Drops may be prepared by pouring the mixture into a mold. Among solid formulations, granules and other powder granules may be prepared by various granulation methods (e.g., extrusion granulation method, crushing granulation method, dry compression granulation method, fluidized-bed granulation method, rolling granulation method, and high-speed stirring granulation method). Tablets can also be prepared by an appropriate combination of, for example, the above granulation method and a tableting method (e.g., wet tableting method) (indirect compression method). Furthermore, capsules can be prepared by filling capsules (soft or hard capsules) with powder granules (e.g., powder or granules) by a common method. The tablets may be coated with sugar or film to prepare sugar- or film-coated tablets. Moreover, the tablets may be in the form of single-layer tablets or layered tablets, such as double-layer tablets. Liquid formulations can be prepared by dissolving or dispersing each component in an aqueous medium (e.g., purified water, heat-purified water, or ethanol-containing purified water) serving as a carrier component, optionally followed by heating, filtration, fabric filtration, or sterilization; and filling a prescribed container with the resulting solution or dispersion, followed by, for example, sterilization.

When the medicinal composition of the present invention is used as a solid formulation, the pyridoxamine salt content of the solid formulation is generally 0.01 to 30 wt%, and preferably 0.1 to 20 wt%. When the medicinal composition of the present invention is used as a liquid formulation, the pyridoxamine salt content of the liquid formulation is generally 0.1 to 20 mg/mL, and preferably 1 to 10 mg/mL.

The dosage and frequency of administration of the medicinal composition of the present invention may be suitably determined depending on various factors, including the dosage form, the age, body weight, and sex of the person who takes the medicinal composition, the number of times of peritoneal dialysis, and other conditions. Generally, the dosage is about 0.1 to 100 mg/kg body weight, and preferably about 0.5 to 50 mg/kg body weight, per day as the amount of pyridoxamine salt, which is an active ingredient. This amount of the medicinal composition is preferably administered about 1 to 4 times a day, depending on the number of times of peritoneal dialysis.

The medicinal composition of the present invention may be administered before or after peritoneal dialysis, but may be preferably before peritoneal dialysis. That is, it is preferable to orally take the medicinal composition of the present invention in advance so that the pyridoxamine salt is introduced into the body, and so that the pyridoxamine or salt thereof is transferred into the blood; and then to intraperitoneally administer (peritoneally dialyze) a peritoneal dialysis fluid containing glucose. Furthermore, the medicinal composition of the present invention may be taken orally after peritoneal dialysis.

The dosing period is a period of time during which patients undergo peritoneal dialysis. The dosing period may be a short period of time (e.g., one day to several weeks), but is generally a long period of time (one year or more).

The medicinal composition of the present invention is used to suppress an increase of carbonyl compounds and/or advanced glycation/lipoxidation end products (AGEs) in the peritoneal cavity and peritoneal tissue of a peritoneal dialysis patient in such a manner that the medicinal composition of the present invention is introduced into the blood by oral administration and passes through the peritoneal tissue from the blood to reach the peritoneal cavity.

The amount of carbonyl compounds in the peritoneal cavity can be analyzed by, for example, the HPLC method described in Experimental Example 2 (3) using 3-deoxyglucosone in the intraperitoneal fluid as an indicator. The amount of carbonyl compounds in the peritoneal tissue can also be analyzed in the same manner using 3-deoxyglucosone in the peritoneal tissue as an indicator. Moreover, the amount of advanced glycation/lipoxidation end products (AGEs) in the peritoneal cavity can be analyzed by, for example, the HPLC method described in Experimental Example 4 (2) using pentosidine in the intraperitoneal fluid as an indicator. The amount of advanced glycation/lipoxidation end products (AGEs) in the peritoneal tissue can also be analyzed in the same manner using pentosidine in the peritoneal tissue as an indicator.

In this case, whether the increase of these compounds is suppressed can be evaluated by comparing the amounts of carbonyl compounds (3-deoxyglucosone) and/or advanced glycation/lipoxidation end products (AGEs) (pentosidine) in the peritoneal cavity and peritoneal tissue when peritoneal dialysis is performed without oral administration of a salt of pyridoxamine (hereinafter referred to as "the control amounts"), and the above amounts when peritoneal dialysis is performed with oral administration of the medicinal composition of the present invention, which comprises a salt of pyridoxamine as an active ingredient (hereinafter referred to as "the test amounts"), as shown in Experimental Examples 2 to 4. The case where the test amounts are lower than the control amounts can be evaluated such that "the increase of carbonyl compounds (3-deoxyglucosone) and/or advanced glycation/lipoxidation end products (AGEs) (pentosidine) in the peritoneal cavity and peritoneal tissue is suppressed."

Moreover, the medicinal composition of the present invention can be used to improve carbonyl stress states in the peritoneal cavity and peritoneal tissue of a peritoneal dialysis patient in such a manner that pyridoxamine or a salt thereof is introduced into the blood by oral administration and passes through the peritoneal tissue from the blood to reach the peritoneal cavity. Furthermore, the medicinal composition of the present invention can be used to protect the peritoneum of a peritoneal dialysis patient from carbonyl stress on the peritoneal tissue in such a manner that pyridoxamine or a salt thereof is introduced into the blood by oral administration and passes through the peritoneal tissue from the blood to reach the peritoneal cavity.

In addition, the medicinal composition of the present invention can be used to suppress or/and improve peritoneal dysfunction or ultrafiltration failure in a peritoneal dialysis patient in such a manner that pyridoxamine or a salt thereof is introduced into the blood by oral administration, and passes through the peritoneal tissue from the blood to reach the peritoneal cavity. The meaning of "improve" in the present invention includes, for example, improving peritoneal dysfunction or ultrafiltration failure, and restoring or activating peritoneal function, particularly ultrafiltration function. Further, the meaning of "suppress" includes, for example, suppressing or reducing the development of peritoneal dysfunction or ultrafiltration failure, suppressing or reducing the aggravation of the disease, or suppressing progression of the disease to alleviate the aggravation of the disease.

### (II) Method for Suppressing Increase of Carbonyl Compounds and/or Advanced Glycation/Lipoxidation End Products (AGEs) in Peritoneal Cavity and Peritoneal Tissue of Peritoneal Dialysis Patient

The present invention also provides a method for suppressing an increase of carbonyl compounds and/or advanced glycation/lipoxidation end products (AGEs) in the peritoneal cavity and peritoneal tissue of a peritoneal dialysis patient after intraperitoneal administration of a glucose-containing peritoneal dialysis fluid.

The meanings of "peritoneal dialysis patient," "glucose-containing peritoneal dialysis fluid," "carbonyl compounds," and "advanced glycation/lipoxidation end products (AGEs)" used herein, and the meaning of "suppress an increase of carbonyl compounds and/or advanced glycation/lipoxidation end products (AGEs) in the peritoneal cavity and peritoneal tissue" and its evaluation method are as explained in section (I) above, and the descriptions thereof are incorporated herein.

The above method of the present invention is characterized by comprising the following steps:
(a) orally administering a pharmaceutically acceptable salt of pyridoxamine to a peritoneal dialysis patient;
(b) intraperitoneally administering a glucose-containing peritoneal dialysis fluid to the patient after and/or before step (a); and
(c) transferring the pyridoxamine or the pharmaceutically acceptable salt thereof into the peritoneal cavity.

The meaning of the "pharmaceutically acceptable salt of pyridoxamine" administered to a peritoneal dialysis patient in step (a) is as explained in section (I) above. The dosage form (oral dosage form) and dosage for oral administration of the salt are also as explained in section (I) above regarding the medicinal composition of the present invention. The descriptions thereof are incorporated herein.

Step (b) is to intraperitoneally administer a glucose-containing peritoneal dialysis fluid to a peritoneal dialysis patient, namely to perform peritoneal dialysis on a peritoneal dialysis patient. This peritoneal dialysis step may be performed before or after the oral administration step (a), but preferably after step (a). That is, in step (a), it is preferable to orally administer a pharmaceutically acceptable salt of pyridoxamine in advance so that the pyridoxamine salt is incorporated into the body, and so that the pyridoxamine or the salt thereof is transferred into the blood; and then to intraperitoneally administer (peritoneally dialyze) a peritoneal dialysis fluid containing glucose. Furthermore, the peritoneal dialysis step (b) may be followed by step (a), i.e., the oral administration of a pyridoxamine salt.

Thus, when peritoneal dialysis treatment is performed preferably in a state where pyridoxamine or a salt thereof is transferred into the blood, the pyridoxamine or the salt thereof in the blood is transferred into the peritoneal cavity via the peritoneal tissue due to the osmotic pressure difference (concentration gradient) between the intraperitoneal and extraperitoneal fluids. This is step (c).

The method of the present invention is used to suppress an increase of carbonyl compounds and/or advanced glycation/lipoxidation end products (AGEs) in the peritoneal cavity and peritoneal tissue of a peritoneal dialysis patient after peritoneal dialysis.

The method for quantitative determination of carbonyl compounds and/or advanced glycation/lipoxidation end products (AGEs) in the peritoneal cavity and peritoneal tissue, and the evaluation of the suppression of the increase of these compounds are as explained in section (I) above, as described above, and the descriptions thereof are incorporated herein.

Moreover, the method of the present invention can be used to improve carbonyl stress states in the peritoneal cavity and peritoneal tissue of a peritoneal dialysis patient. Furthermore, the method of the present invention can be used to protect the peritoneum of a peritoneal dialysis patient from carbonyl stress on the peritoneal tissue.

In this case, the method comprises, in addition to the above steps (a) to (c), the following step (d):
(d) suppressing an increase of carbonyl compounds and/or advanced glycation/lipoxidation end products (AGEs) in the peritoneal cavity and peritoneal tissue caused by the intraperitoneal administration of the glucose-containing peritoneal dialysis fluid.

### (III) Method for Protecting Peritoneal Tissue of Peritoneal Dialysis Patient from Carbonyl Stress Caused by Glucose-containing Peritoneal Dialysis

That is, the present invention also provides a method for protecting the peritoneal tissue of a peritoneal dialysis patient from carbonyl stress caused by glucose-containing peritoneal dialysis.

The meanings of "peritoneal dialysis patient" and "glucose-containing peritoneal dialysis fluid" used herein are as explained in section (I) above, and the descriptions thereof are incorporated herein.

The above method of the present invention is characterized by comprising the following steps:
(a) orally administering a pharmaceutically acceptable salt of pyridoxamine to a peritoneal dialysis patient;
(b) intraperitoneally administering a glucose-containing peritoneal dialysis fluid to the patient after and/or before step (a);
(c) transferring the pyridoxamine or the pharmaceutically acceptable salt thereof into the peritoneal cavity; and
(d) suppressing an increase of carbonyl compounds and/or advanced glycation/lipoxidation end products (AGEs) in the peritoneal cavity and peritoneal tissue caused by the intraperitoneal administration of the glucose-containing peritoneal dialysis fluid.

Among these steps, steps (a) to (c) are as explained in section (II) above, and the descriptions thereof are incorporated herein.

When a glucose-containing peritoneal dialysis fluid is intraperitoneally administered (peritoneally dialyzed) to a peritoneal dialysis patient without oral administration of a salt of pyridoxamine, as described above, osmotic pressure difference (concentration gradient) occurs between the intraperitoneal and extraperitoneal fluids. Thereby, carbonyl compounds present in the blood and extraperitoneal cavity flow into the peritoneal cavity via the peritoneal tissue and are accumulated in the intraperitoneal fluid. Further, when the glucose-containing peritoneal dialysis fluid contains carbonyl compounds, such exogenous carbonyl compounds flow into the intraperitoneal fluid by peritoneal dialysis.

According to the above method, steps (a) to (c) allow the pyridoxamine salt to circulate in the blood and be transferred into the intraperitoneal fluid. As a result, overall carbonyl compounds present in the body, including the peritoneal cavity, can be eliminated, and thereby the increase of carbonyl compounds in the peritoneal cavity and peritoneal tissue caused by the above phenomenon can be suppressed. This also allows suppression of an increase of advanced glycation/lipoxidation end products (AGEs) produced by the reaction of carbonyl compounds with proteins. This is step (d).

In this case, whether the increase of carbonyl compounds and/or AGEs is suppressed can be evaluated by the method explained in section (I). More specifically, it can be evaluated by comparing the amounts of carbonyl compounds (3-deoxyglucosone) and/or AGEs (pentosidine) in the peritoneal cavity and peritoneal tissue when peritoneal dialysis is performed without oral administration of a salt of pyridoxamine (hereinafter referred to as "the control amounts") and the above amounts when peritoneal dialysis is performed with oral administration of a salt of pyridoxamine (hereinafter referred to as "the test amounts"), as shown in Experimental Examples 2 to 4. The case where the test amounts are lower than the control amounts can be evaluated such that "the increase of carbonyl compounds (3-deoxyglucosone) and/or advanced glycation/lipoxidation end products (AGEs) (pentosidine) in the peritoneal cavity and peritoneal tissue is suppressed."

AGEs (pentosidine) can simultaneously serve as an indicator of tissue degeneration caused by carbonyl stress. Whether the peritoneal tissue is protected from carbonyl stress can be evaluated by measuring the amount of AGEs (pentosidine) in the peritoneal tissue.

More specifically, as shown in Experimental Example 4, it can be evaluated by comparing the amount of AGEs (pentosidine) in the peritoneal tissue when peritoneal dialysis is performed without oral administration of a salt of pyridoxamine (hereinafter referred to as "the control amount") and the above amount when peritoneal dialysis is performed with oral administration of a salt of pyridoxamine (hereinafter referred to as "the test amount"). The case where the test amount is lower than the control amount can be evaluated such that the peritoneal tissue is protected from carbonyl stress.

The method of the present invention is used to suppress or/and improve peritoneal dysfunction or ultrafiltration failure in peritoneal dialysis patients. The meanings of "suppressing" and "improving" peritoneal dysfunction or ultrafiltration failure as used herein are as explained in section (I), and the descriptions thereof are incorporated herein.

### (IV) Pharmaceutically Acceptable Salt of Pyridoxamine Having Oral Dosage Form

The present invention also provides a pharmaceutically acceptable salt of pyridoxamine having an oral dosage form for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

The meanings of "peritoneal dialysis patient," "glucose-containing peritoneal dialysis fluid," "carbonyl compounds," and "advanced glycation/lipoxidation end products (AGEs)" used herein; the meaning of "suppress an increase of carbonyl compounds and/or advanced glycation/lipoxidation end products (AGEs) in the peritoneal cavity and peritoneal tissue" and its evaluation method; and the meaning, type, dosage form, dosage, and administration route of the "pharmaceutically acceptable salt of pyridoxamine" are as explained in section (I) above, and the descriptions thereof are incorporated herein.

The "pharmaceutically acceptable salt of pyridoxamine having an oral dosage form" of the present invention can be used to improve carbonyl stress states in the peritoneal cavity and peritoneal tissue of a peritoneal dialysis patient in such a manner that pyridoxamine or a salt thereof is introduced into the blood by oral administration, and passes through the peritoneal tissue from the blood to reach the peritoneal cavity. Moreover, the "pharmaceutically acceptable salt of pyridoxamine having an oral dosage form" of the present invention can be used to protect the peritoneum of a peritoneal dialysis patient from carbonyl stress on the peritoneal tissue in such a manner that pyridoxamine or a salt thereof is introduced into the blood by oral administration, and passes through the peritoneal tissue from the blood to reach the peritoneal cavity.

Furthermore, the "pharmaceutically acceptable salt of pyridoxamine having an oral dosage form" of the present invention can be used to suppress or/and improve peritoneal dysfunction or ultrafiltration failure in a peritoneal dialysis patient in such a manner that pyridoxamine or a salt thereof is introduced into the blood by oral administration and passes through the peritoneal tissue from the blood to reach the peritoneal cavity. The meanings of "suppress" and "improve" used herein are also as explained in section (I) above, and the descriptions thereof are incorporated herein.

### (V) Use of Pharmaceutically Acceptable Salt of Pyridoxamine

The present invention provides use of a pharmaceutically acceptable salt of pyridoxamine for producing an oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

The meanings of "peritoneal dialysis patient," "glucose-containing peritoneal dialysis fluid," "carbonyl compounds," and "advanced glycation/lipoxidation end products (AGEs)" used herein; the meaning of "suppress an increase of carbonyl compounds and/or advanced glycation/lipoxidation end products (AGEs) in the peritoneal cavity and peritoneal tissue" and its evaluation method; and the meaning, type, dosage form, dosage, and administration route of the "pharmaceutically acceptable salt of pyridoxamine" are as explained in section (I) above, and the descriptions thereof are incorporated herein.

The medicinal composition targeted by the present invention can be used to improve carbonyl stress states in the peritoneal cavity and peritoneal tissue of a peritoneal dialysis patient in such a manner that pyridoxamine or a salt thereof is introduced into the blood by oral administration and passes through the peritoneal tissue from the blood to reach the peritoneal cavity. Moreover, the medicinal composition targeted by the present invention can be used to protect the peritoneum of a peritoneal dialysis patient from carbonyl stress on the peritoneal tissue in such a manner that pyridoxamine or a salt thereof is introduced into the blood by oral administration, and passes through the peritoneal tissue from the blood to reach the peritoneal cavity.

Furthermore, the medicinal composition targeted by the present invention can be used to suppress or/and improve peritoneal dysfunction or ultrafiltration failure in a peritoneal dialysis patient in such a manner that pyridoxamine or a salt thereof is introduced into the blood by oral administration, and passes through the peritoneal tissue from the blood to reach the peritoneal cavity. The meanings of "suppress" and "improve" used herein are also as explained in section (I), and the descriptions thereof are incorporated herein.

### Examples

The present invention is described below based on Experimental Examples and Examples. However, the present invention is not limited to these Experimental Examples and Examples, and can be suitably modified using conventionally known techniques within a range that does not deviate from the scope of the present invention.

### Experimental Example 1: Stability of Pyridoxamine in Peritoneal Dialysis Fluid

### (A) Test Procedures

### (1) Test Materials

- Peritoneal dialysis fluid: Dianeal (registered trademark)-N PD-4-1.5 (Baxter Limited)
- Pyridoxamine dihydrochloride (Wako Pure Chemical Industries, Ltd.)

The above peritoneal dialysis fluid is composed of an acid solution containing glucose (pH 3.5 to 4.5) and an alkaline solution containing an electrolyte, such as sodium lactate (pH 7.0 to 7.7), which are respectively placed in upper and lower compartments of a two-compartment container divided by an openable partition. When peritoneal dialysis treatment is performed on a chronic renal failure patient, the partition is opened between the upper and lower compartments so that both solutions are mixed, and the mixture is used after its pH is adjusted to a neutral range (pH 6.5 to 7.5). Table 1 shows the compositions of the solutions in the upper and lower compartments before mixing, and the composition of the mixture of these solutions.

**Table 1**

| | Dianeal-N PD-4 1.5 peritoneal dialysis fluid | | | | |
|---|---|---|---|---|---|
| Upper compartment | 362 mL | 544 mL | 725 mL | 906 mL | 1812 mL |

| **Active ingredient** | | | | | |
|---|---|---|---|---|---|
| Glucose (C₆H₁₂O₆) | 13.60 g | 20.40 g | 27.20 g | 34.00 g | 68.00 g |
| Calcium chloride (CaCl₂·2H₂O) | 183.0 mg | 274.5 mg | 366.0 mg | 457.5 mg | 915.0 mg |
| Magnesium chloride (MgCl₂·6H₂O) | 50.8 mg | 76.2 mg | 101.6 mg | 127.0 mg | 254.0 mg |
| **Additive** | | | | | |
| Sodium hydroxide (NaOH) (pH adjuster) | Adequate amount | Adequate amount | Adequate amount | Adequate amount | Adequate amount |
| Hydrochloric acid (HCl) (pH adjuster) | Adequate amount | Adequate amount | Adequate amount | Adequate amount | Adequate amount |
| Lower compartment | 638 mL | 956 mL | 1275 mL | 1594 mL | 3188 mL |

| **Active ingredient** | | | | | |
|---|---|---|---|---|---|
| Sodium lactate solution (C₃H₅NaO₃) (as sodium lactate) | 4.480 g | 6.720 g | 8.960 g | 11.200 g | 22.400 g |
| Sodium chloride (NaCl) | 5.380 g | 8.070 g | 10.760 g | 13.450 g | 26.900 g |

| **Additive** | | | | | |
|---|---|---|---|---|---|
| L-histidine (C₆H₉N₃O₂) (stabilizer) | 58.9 mg | 88.35 mg | 117.8 mg | 147.25 mg | 294.5 mg |
| Sodium hydroxide (NaOH) (pH adjuster) | Adequate amount | Adequate amount | Adequate amount | Adequate amount | Adequate amount |
| Hydrochloric acid (HCl) (pH adjuster) | Adequate amount | Adequate amount | Adequate amount | Adequate amount | Adequate amount |
| After mixing | 1000 mL | 1500 mL | 2000 mL | 2500 mL | 5000 mL |

| **Component and amount (w/v%)** | | | | | |
|---|---|---|---|---|---|
| Glucose (C₆H₁₂O₆) | 1.36 | | | | |
| Calcium chloride (CaCl₂·2H₂O) | 0.0183 | | | | |
| Magnesium chloride (MgCl₂·CH₂O) | 0.00508 | | | | |
| Sodium lactate (C₃H₅NaO₃) | 0.448 | | | | |
| Sodium chloride (NaCl) | 0.538 | | | | |

| **Electrolyte concentration (mEq/L)** | | | | | |
|---|---|---|---|---|---|
| Na⁺ | 132 | | | | |
| Ca²⁺ | 2.5 | | | | |
| Mg²⁺ | 0.5 | | | | |
| Cl⁻ | 95 | | | | |
| Lactic acid ion | 40 | | | | |

### (2) Experimental Conditions

The glucose-containing solution (pH 4.15) in the upper compartment of the peritoneal dialysis fluid bag, the electrolyte solution (pH 7.02) in the lower compartment, and the mixture of the upper and lower compartment solutions (pH 6.76) were used as solvents, and 5 µg/ml of pyridoxamine dihydrochloride was dissolved in each solvent to obtain test samples. The test samples were left for three weeks at room temperature (25±5°C), which was almost the same condition that renal failure patients generally store. During that period, sampling was conducted with time, and the amount of pyridoxamine contained in each test sample was measured.

The amount of pyridoxamine contained in the test samples was measured by high-performance liquid chromatography (HPLC) under the following conditions.

### HPLC Conditions

High-performance liquid chromatography: Shimadzu Corporation
Mobile phase: 0.1% trifluoroacetic acid-containing aqueous solution
Stationary phase: C18 column (Develosil) , 4.6 x 150 mm, 5 µm; Nomura Chemical Co., Ltd.)
Flow rate: 0.8 mL/min
Detection: UV detection of absorbance at a wavelength of 294 nm (UV detector: Shimadzu Corporation)

### (B) Results

The experimental results are shown in Table 2. In Table 2, the upper numerical value in each column shows the peak height of pyridoxamine measured by HPLC (measurement value on chromatogram), and the lower numerical value in parentheses shows the rate (%) of the peak height of pyridoxamine on each measurement day to the peak height of pyridoxamine on the first measurement day of each solution (100%).

**Table 2**

| Day | 1st | 2nd | 6th | 7th | 8th | 10th | 15th | 21st |
|---|---|---|---|---|---|---|---|---|
| Time | 0 | 24 | 120 | 144 | 168 | 216 | 336 | 480 |
| Peritoneal dialysis fluid upper-compartment solution (pH 4.15) | 14051 (100) | 13271 (94) | 13084 (93) | 13316 (95) | 11561 (82) | 12139 (86) | 11518 (82) | 10605 (75) |
| Peritoneal dialysis fluid lower-compartment solution (pH 7.02) | 13265 (100) | 12959 (98) | 13029 (98) | 12800 (96) | 12206 (92) | 11887 (90) | 11342 (86) | 10505 (79) |
| Peritoneal dialysis fluid mixture (pH 6.76) | 11061 (100) | 9991 (90) | 10323 (93) | 9821 (89) | 9924 (90) | 9612 (87) | 9429 (85) | 6322 (57) |

As shown in Table 2, it was confirmed that when the solutions in the upper and lower compartments of the peritoneal dialysis fluid bag, and the mixture of the upper- and lower-compartment solutions were left for three weeks, the pyridoxamine content of all the solutions decreased with time. This revealed that regardless of the type of solution (i.e., upper-compartment solution, lower-compartment solution, and mixture thereof), pyridoxamine was unstable in the peritoneal dialysis fluid, even in the acid solution in the upper compartment. Accordingly, to commercialize a peritoneal dialysis fluid containing pyridoxamine, even in the case of a two-compartment structure type, it is necessary to stabilize pyridoxamine in the peritoneal dialysis fluid by, for example, using a stabilizer or improving the dialysis fluid bag.

### Experimental Example 2: Evaluation of Transfer of Pyridoxamine into Peritoneal Cavity by Oral Administration of Pyridoxamine Salt, and Carbonyl Stress State in Peritoneal Cavity

Pyridoxamine dihydrochloride was orally administered to uremic rat models with kidney subtotal excision, and transfer of pyridoxamine into the peritoneal cavity was evaluated. Further, using the above uremic rat models, the amount of dicarbonyl compounds (3-deoxyglucosone) in the intraperitoneal fluid was measured to evaluate a carbonyl stress state.

### (A) Test Procedures

### (1) Production of Uremic Animal Models and Collection of Test Samples (Blood and Intraperitoneal Fluid)

The uremic rat models were produced using 7-week-old (body weight: 300 g or less) Sprague-Dawley male rats (CLEA Japan, Inc.) by the method described in NPL 27 and NPL 28. More specifically, the above rats were first subjected to 1/3 nephrectomy under anesthesia, and then to 1/2 nephrectomy after a week, thereby producing 5/6 nephrectomy rats (rats with kidney subtotal excision). These rats were allowed to freely take a standard diet for experimental rats (containing 1.06% calcium, 0.9% phosphate, and 21% protein; CE2: CLEA Japan, Inc.) and tap water until the eve of slaughter.

The kidney subtotal excision rats with chronic renal failure (uremic rat models) produced in the above manner were separated into two groups (each group: n = 3). Distilled water for injection (Otsuka Pharmaceutical Co., Ltd.; here and below) was orally administered compulsorily to Group 1 (control group) once a day, while a pyridoxamine salt aqueous solution prepared by dissolving 30 mg of pyridoxamine dihydrochloride (Sigma; here and below) in 2 mL of distilled water for injection was orally administered compulsorily to Group 2 (pyridoxamine salt orally administered group) once a day. On the 6th day of administration, after distilled water for injection and the pyridoxamine salt aqueous solution were orally administered, respectively, to Groups 1 and 2, 20 ml of a peritoneal dialysis fluid (pH 4.5 to 5.5) (trade name: Dianeal (registered trademark) PD-4 4.25; Baxter Limited) was injected intraperitoneally to both groups.

Table 3 shows the composition of the peritoneal dialysis fluid.

**Table 3**

| Component and Amount (w/v%) | | |
|---|---|---|
| Component | Chemical formula | Amount |
| Glucose | C₆H₁₂O₆ | 3.86 |
| Sodium chloride | NaCl | 0.538 |
| Sodium lactate | C₃H₅NaO₃ | 0.448 |
| Calcium chloride | CaCl₂-2H₂O | 0.0183 |
| Magnesium chloride | MgCl₂·6H₂O | 0.00508 |

| Electrolyte concentration | | |
|---|---|---|
| Electrolyte | Chemical formula | Concentration (mEq/L) |
| Sodium ion | Na⁺ | 132 |
| Calcium ion | Ca²⁺ | 2.5 |
| Magnesium ion | Mg²⁺ | 0.5 |
| Chlorine ion | Cl⁻ | 95 |
| Lactic acid ion | C₃H₅O₃⁻ | 40 |

Subsequently, on the 7th day of administration, as normal, distilled water for injection was orally administered compulsorily to Group 1, and the pyridoxamine salt aqueous solution was orally administered compulsorily to Group 2. Within an hour after administration, the rats were killed by removing blood, and the blood and intraperitoneal fluid were collected as test samples (specimens). The serum and intraperitoneal fluid were placed in containers for individual rats and frozen at -80°C before use in the measurement.

When the quantitative analysis of pyridoxamine and dicarbonyl compounds (3-deoxyglucosone) in the samples was performed by liquid chromatography (HPLC), the above frozen test samples (serum and intraperitoneal fluid) were used after natural thawing at room temperature.

### (2) Quantitative Analysis of Pyridoxamine in Blood and Intraperitoneal Fluid by HPLC

### Pretreatment of Test Samples

Before being subjected to HPLC, the blood and intraperitoneal fluid collected in the above manner were pretreated by the following procedure.

First, 50 µL of each test sample (serum and intraperitoneal fluid) was taken in a test tube. To the test tube, 100 µL of acetonitrile (Wako Pure Chemical Ind., Ltd.) was added and well stirred, and the resulting mixture was left at room temperature for 10 minutes. Subsequently, the mixture was subjected to centrifugation at 12,000 rpm at 4°C for 10 minutes to precipitate proteins, and the supernatant was then collected for use as a sample for pyridoxamine quantitative analysis by HPLC.

### Quantitative Analysis of Pyridoxamine

The quantitative analysis of pyridoxamine was carried out under the HPLC conditions described in Experimental Example 1 (2). The quantitative analysis was performed using a standard calibration curve previously prepared by measurement under the above HPLC conditions using standard solutions of pyridoxamine of known concentrations.

### (3) Quantitative Analysis of Dicarbonyl Compounds in Intraperitoneal Fluid by HPLC

### Pretreatment of Test Sample

Before being subjected to HPLC, the intraperitoneal fluid collected in the above manner was pretreated by the following procedure.

First, the test sample (intraperitoneal fluid) was subjected to centrifugation at 10,000 rpm at 4°C for 10 minutes, and the supernatant was collected. Separately, 308 µL of a solution prepared by reacting 0.2% o-phenylenediamine, 1 µM 2,3-butanedione, and 0.7 M perchloric acid in a darkroom at room temperature for 30 minutes was added to 140 µL of the above test sample supernatant. After mixing by inversion, the mixture was reacted in a darkroom at room temperature for 3 hours. The resultant was subjected again to centrifugation (10,000 rpm, 10 minutes, 4°C), and the supernatant was collected for use as an HPLC sample.

### Quantitative Analysis of Dicarbonyl Compounds

The quantitative analysis of dicarbonyl compounds (3-deoxyglucosone) was performed by reversed-phase high-performance liquid chromatography (HPLC) under the following conditions. The quantitative analysis was performed using a standard calibration curve previously prepared by measurement under the following HPLC conditions using standard solutions of 3-deoxyglucosone of known concentrations.

### HPLC Conditions

High-performance liquid chromatography: Shimadzu Corporation
Mobile phase: Using Eluent A (0.1% trifluoroacetic acid) and Eluent B (80% acetonitrile containing 0.08% trifluoroacetic acid), the concentration of Eluent B was changed from 20% to 28% over 25 minutes.

Stationary phase: C18 column (Puresil, 4.6 x 150 mm, 5 µm; Nihon Waters K.K.)
Flow rate: 0.8 mL/min
Detection: UV detection of absorbance at a wavelength of 315 nm (UV detector: Shimadzu Corporation)
Sample volume: 20 µL

### (B) Results

### (1) Transfer of Pyridoxamine into Peritoneal Cavity

Tables 4 and 5 respectively show the concentration of pyridoxamine in the serum, and the concentration and amount of pyridoxamine in the intraperitoneal fluid, measured in each of the uremic rat model groups (Group 1: control group; Group 2: pyridoxamine salt orally administered group). The pyridoxamine amount shown in Table 5 is the amount (µg) of pyridoxamine contained in 20.8 ml of the collected intraperitoneal fluid.

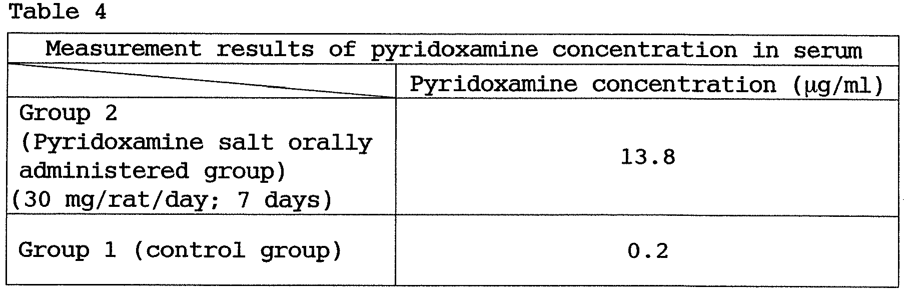

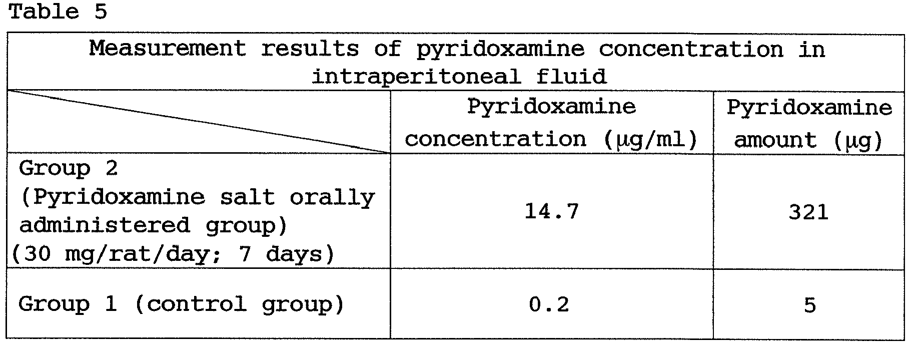

As shown in Tables 4 and 5, it was confirmed that when pyridoxamine dihydrochloride was orally administered to the uremic rat models, the pyridoxamine was transferred into the blood and peritoneal cavity. In particular, as shown in Table 5, the pyridoxamine concentration in the intraperitoneal cavity of the uremic rat models to which a pyridoxamine salt had been orally administered (Group 2) was higher by 65 times or more than that of the uremic rat models to which a pyridoxamine salt had not been orally administered (Group 1).

This confirmed that when a salt of pyridoxamine was orally administered to the uremic rat models, the pyridoxamine was efficiently and stably transferred into the blood, and also transferred into the peritoneal cavity while moving from the blood three-dimensionally and vertically through the peritoneal tissue.

### (2) Quantitative Analysis of Dicarbonyl Compounds in Intraperitoneal Fluid

Table 6 shows the concentration of dicarbonyl compounds (3-deoxyglucosone) in the intraperitoneal fluid measured in each of the uremic rat model groups (Group 1: control group; Group 2: pyridoxamine salt orally administered group).

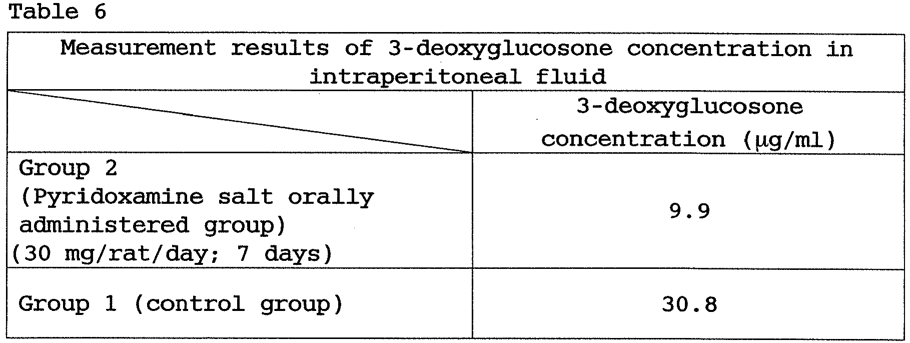

As shown in Table 6, the concentration of dicarbonyl compounds (3-deoxyglucosone) in the peritoneal cavity of the uremic rat models that had undergone peritoneal dialysis with oral administration of a pyridoxamine salt (Group 2) was less than one-third of that of the uremic rat models that had undergone peritoneal dialysis without oral administration of a pyridoxamine salt (Group 1).

The results of this Experimental Example confirmed that the pyridoxamine salt administered as an oral drug to the uremic rat models was transferred into the blood (Table 4), and further transferred into the peritoneal cavity while moving from the blood three-dimensionally and vertically through the peritoneal tissue (Table 5). It was also confirmed that when a salt of pyridoxamine was orally administered to the uremic rat models, the concentration of dicarbonyl compounds (3-deoxyglucosone) in the peritoneal cavity, which would have increased by peritoneal dialysis without oral administration of a salt of pyridoxamine, was greatly reduced; and that the carbonyl stress state in the peritoneal cavity caused by peritoneal dialysis was thereby significantly improved (Table 6).

These facts demonstrate that the orally administered pyridoxamine in the form of a salt was transferred into the blood, and further transferred into the peritoneal cavity from the blood, thereby suppressing and improving the carbonyl stress state in the peritoneal cavity caused by peritoneal dialysis.

### Experimental Example 3: Evaluation of Carbonyl Stress State in Peritoneal Cavity after Oral Administration of Pyridoxamine Salt (II)

Kidney subtotal excision rats with chronic renal failure (uremic rat models) were produced in the same manner as in Experimental Example 2. Distilled water for injection was orally administered compulsorily to Group 1 (control group) (n = 3) once a day, and the pyridoxamine salt aqueous solution was orally administered compulsorily to Group 2 (pyridoxamine salt orally administered group) once a day. On the 6th day of administration, after distilled water for injection and the pyridoxamine salt aqueous solution were orally administered, respectively, to Groups 1 and 2, 20 ml of a neutral peritoneal dialysis fluid (pH 6.5 to 7.5) to be mixed before use (trade name: Dianeal (registered trademark)-N PD-4-1.5 mixture; Baxter Limited) (Table 1) was injected intraperitoneally to both groups, in place of the acid peritoneal dialysis fluid administered in Experimental Example 2. As explained in Experimental Example 1, the peritoneal dialysis fluid was composed of an upper-compartment solution and a lower-compartment solution separated by a partition, and both solutions were mixed immediately before being injected into the peritoneal cavity.

Subsequently, as in Experimental Example 2, on the 7th day of administration, as normal, distilled water for injection was orally administered compulsorily to Group 1, and the pyridoxamine salt aqueous solution was orally administered compulsorily to Group 2. Within an hour after administration, the rats were killed by removing blood, and the intraperitoneal fluid was collected as a test sample (specimen).

The concentration of dicarbonyl compounds (3-deoxyglucosone) in the intraperitoneal fluid of each group was measured by HPLC in the same manner as in Experimental Example 2. The results are shown in Fig. 7.

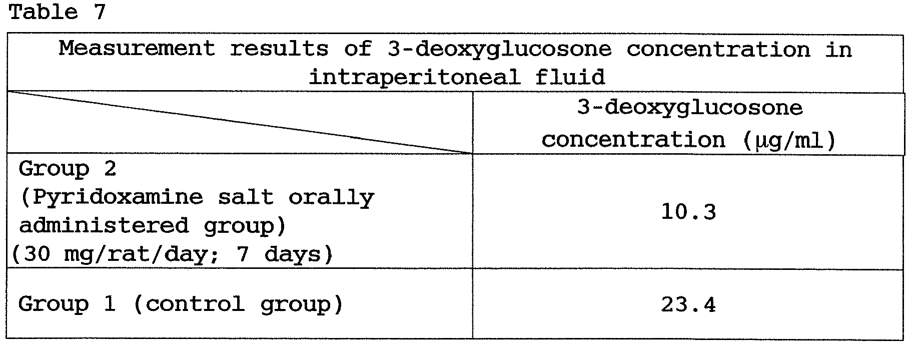

As shown in Table 7, the concentration of dicarbonyl compounds (3-deoxyglucosone) in the peritoneal cavity of the uremic rat models that had undergone peritoneal dialysis with oral administration of a pyridoxamine salt (Group 2) was less than one-half of that of the uremic rat models that had undergone peritoneal dialysis without oral administration of a pyridoxamine salt (Group 1).

As compared with the acid peritoneal dialysis fluid used in Experimental Example 2, the peritoneal dialysis fluid to be mixed before use filled in a two-compartment package bag is less likely to produce carbonyl compounds in the production process, and the peritoneal dialysis fluid itself contains little carbonyl compounds. However, as shown in Table 7, even when peritoneal dialysis was performed using such a mixed-before-use-type peritoneal dialysis fluid, a high concentration of carbonyl compounds was detected in the intraperitoneal fluid (see the results of Group 1). The cause of this is considered to be that the osmotic pressure difference between the intraperitoneal fluid and the body fluid resulting from peritoneal dialysis allows endogenous carbonyl compounds to move from the outside of the peritoneal cavity into the peritoneal cavity, thereby contributing to the generation of carbonyl stress states in the peritoneal tissue and peritoneal cavity.

As shown in the above experiment, when a salt of pyridoxamine is orally administered to the uremic rat models, endogenous carbonyl compounds are eliminated, and the increase of endogenous carbonyl compounds in the peritoneal cavity due to transfer of endogenous carbonyl compounds from the outside of the peritoneal cavity into the peritoneal cavity caused by peritoneal dialysis can be suppressed. As a result, the carbonyl stress state in the peritoneal cavity caused by endogenous carbonyl compounds can be improved.

### Experimental Example 4: Evaluation of Peritoneum Protective Effect by Oral Administration of Pyridoxamine Salt

The protective effect of oral administration of a pyridoxamine salt on the deterioration of the peritoneum caused by peritoneal dialysis was evaluated. More specifically, uremic rat models were subjected to intraperitoneal administration of a peritoneal dialysis fluid together with oral administration of a pyridoxamine salt, and the concentration of pentosidine in the peritoneal tissue, which was an indicator of tissue degeneration caused by enhanced carbonyl stress, was measured. Pentosidine is an advanced glycation/lipoxidation end product (AGE).

### (A) Test Procedures

### (1) Preparation of Uremic Animal Models and Collection of Test Sample

Kidney subtotal excision rats were produced in the same manner as in Experimental Example 2. Distilled water for injection was orally administered compulsorily to Group 1 (control group) (n = 3) once a day, and the pyridoxamine salt aqueous solution was orally administered compulsorily to Group 2 (pyridoxamine salt orally administered group) once a day. These rats developed symptoms of uremia due to chronic renal failure on the 5th day of administration. Using them as uremic rat models, on the 6th day of administration, after distilled water for injection and the pyridoxamine salt aqueous solution were orally administered, respectively, to Groups 1 and 2, 20 ml of the acid peritoneal dialysis fluid (pH 4.5 to 5.5) (trade name: Dianeal (registered trademark) PD-44.25; Baxter Limited) (Table 3), which was used in Experimental Example 2, was injected intraperitoneally to both groups. Subsequently, as in Experimental Example 2, on the 7th day of administration, as normal, distilled water for injection was orally administered compulsorily to Group 1, and the pyridoxamine salt aqueous solution was orally administered compulsorily to Group 2. Within an hour after administration, the rats were killed by removing blood, followed by laparotomy, and the peritoneum was collected as a test sample.

The collected peritoneum was taken in a test tube in an amount of about 50 mg to 80 mg. After degreasing for 16 hours using a mixture of chloroform and methanol (2:1), the resultant was washed well with methanol, and dried to solid under suction and negative pressure. To the thus-prepared dried peritoneal product, 6N hydrochloric acid was added, and hydrolysis was performed at 110°C for 24 hours. After hydrolysis, concentration and drying was performed using a centrifugal concentrator. The resulting dried product was dissolved in purified water, filtered through a pore filter with 0.5-µm holes, and diluted to any concentration for use as a sample for pentosidine measurement.

### (2) HPLC Conditions for Measurement of Pentosidine Concentration

The sample for pentosidine measurement prepared in the above manner was subjected to HPLC under the following conditions, and pentosidine contained in the peritoneum was quantitatively analyzed. The quantitative analysis was performed using a standard calibration curve previously prepared by measurement under the following HPLC conditions using standard solutions of synthetic pentosidine of known concentrations.

### HPLC Conditions

High-performance liquid chromatography: Shimadzu Corporation
Mobile phase: Eluent A (0.1% trifluoroacetic acid) and Eluent B (80% acetonitrile containing 0.08% trifluoroacetic acid) were used. Eluent A and Eluent B were mixed at a rate of 1 mL/min over 30 minutes, and the elution was performed with a gradient of acetonitrile concentration.
Stationary phase: C18 column (Puresil, 4.6 x 250 mm, 5 µm: Nihon Waters K.K.)
Flow rate: 0.8 mL/min
Detection: Fluorescence detection (excitation wavelength: 335 nm/absorption wavelength: 385 nm) (fluorescence detector: Shimadzu Corporation)
Sample volume: 20 µL

### (B) Results

The results are shown in Fig. 8. The pentosidine concentration in the peritoneum tissue is a concentration of pentosidine per mg of peritoneum (wet mass).

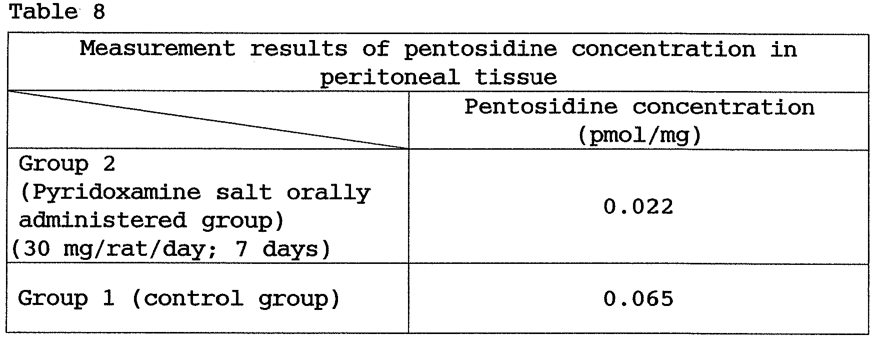

As shown in Table 8, the pentosidine concentration in the peritoneum tissue of the uremic rat models that had undergone peritoneal dialysis with oral administration of a pyridoxamine salt (Group 2) was significantly lower than the pentosidine concentration in the peritoneum tissue of the uremic rat models that had undergone peritoneal dialysis without oral administration of a pyridoxamine salt (Group 1). As described above, pentosidine is a marker compound serving as an indicator of tissue degeneration caused by enhanced carbonyl stress (NPL 9 to NPL 14). This indicated that the degeneration of the peritoneal tissue of the uremic rat models of Group 2 caused by enhanced carbonyl stress was inhibited by oral administration of a pyridoxamine salt; in other words, the peritoneum was protected from carbonyl stress by oral administration of a pyridoxamine salt.

Moreover, this fact proves that the pyridoxamine orally administered in the form of a salt improves the carbonyl stress state in the peritoneal tissue while the pyridoxamine salt is transferred into the blood, and moves three-dimensionally and vertically through the peritoneal tissue from the blood.

The above results revealed that the present invention can effectively suppress a decline in the peritoneal function of patients who undergo long-term peritoneal dialysis (e.g., chronic renal failure patients) caused by peritoneal dialysis, by eliminating carbonyl compounds transferred from the blood in the body into the peritoneal cavity while moving vertically through the peritoneal tissue, and by inhibiting the production and accumulation of pentosidine (AGEs), thereby improving the carbonyl stress state in the peritoneal cavity.

### Industrial Applicability

According to the present invention, as simple a method as oral administration can more effectively suppress functional decline in the peritoneum and its neighboring tissue.

## Claims

1. An oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid, the oral medicinal composition comprising a pharmaceutically acceptable salt of pyridoxamine as an active ingredient.

2. The oral medicinal composition according to claim 1, wherein the carbonyl compound is 3-deoxyglucosone.

3. The oral medicinal composition according to claim 1 or 2, wherein the advanced glycation/lipoxidation end product (AGE) is pentosidine.

4. The oral medicinal composition according to any one of claims 1 to 3, which is a medicinal composition for improving a state of carbonyl stress in the peritoneal cavity and peritoneal tissue.

5. The oral medicinal composition according to any one of claims 1 to 3, which is a medicinal composition for protecting the peritoneum of a peritoneal dialysis patient.

6. The oral medicinal composition according to any one of claims 1 to 5, which is for suppressing or/and improving peritoneal dysfunction or ultrafiltration failure in peritoneal dialysis patients.

7. A method for suppressing an increase of carbonyl compounds and/or advanced glycation/lipoxidation end products (AGEs) in the peritoneal cavity and peritoneal tissue of a peritoneal dialysis patient after intraperitoneal administration of a glucose-containing peritoneal dialysis fluid, the method comprising the steps of:
(a) orally administering a pharmaceutically acceptable salt of pyridoxamine to the peritoneal dialysis patient;
(b) intraperitoneally administering a glucose-containing peritoneal dialysis fluid to the patient after and/or before step (a); and
(c) transferring the pyridoxamine or the pharmaceutically acceptable salt thereof into the peritoneal cavity.

8. The method according to claim 7, wherein the carbonyl compound is 3-deoxyglucosone.

9. The method according to claim 7 or 8, wherein the advanced glycation/lipoxidation end product (AGE) is pentosidine.

10. A method for protecting the peritoneal tissue of a peritoneal dialysis patient from carbonyl stress caused by glucose-containing peritoneal dialysis, the method comprising the steps of:
(a) orally administering a pharmaceutically acceptable salt of pyridoxamine to the peritoneal dialysis patient;
(b) intraperitoneally administering a glucose-containing peritoneal dialysis fluid to the patient after and/or before step (a);
(c) transferring the pyridoxamine or the pharmaceutically acceptable salt thereof into the peritoneal cavity; and
(d) suppressing an increase of carbonyl compounds and/or advanced glycation/lipoxidation end products (AGEs) in the peritoneal cavity and peritoneal tissue caused by the intraperitoneal administration of the glucose-containing peritoneal dialysis fluid.

11. The method according to claim 10, wherein the carbonyl compound is 3-deoxyglucosone.

12. The method according to claim 10 or 11, wherein the advanced glycation/lipoxidation end product (AGE) is pentosidine.

13. The method according to any one of claims 7 to 12, which is used to suppress or/and improve peritoneal dysfunction or ultrafiltration failure in peritoneal dialysis patients.

14. A pharmaceutically acceptable salt of pyridoxamine having an oral dosage form for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

15. The pharmaceutically acceptable salt of pyridoxamine according to claim 14, wherein the carbonyl compound is 3-deoxyglucosone.

16. The pharmaceutically acceptable salt of pyridoxamine according to claim 14 or 15, wherein the advanced glycation/lipoxidation end product (AGE) is pentosidine.

17. The pharmaceutically acceptable salt of pyridoxamine according to any one of claims 14 to 16, which is for improving a state of carbonyl stress in the peritoneal cavity and peritoneal tissue of a peritoneal dialysis patient.

18. The pharmaceutically acceptable salt of pyridoxamine according to any one of claims 14 to 16, which is for protecting the peritoneum of a peritoneal dialysis patient.

19. The pharmaceutically acceptable salt of pyridoxamine according to any one of claims 14 to 18, which is for suppressing or/and improving peritoneal dysfunction or ultrafiltration failure in peritoneal dialysis patients.

20. Use of a pharmaceutically acceptable salt of pyridoxamine for producing an oral medicinal composition for peritoneal dialysis patients to suppress an increase of carbonyl compounds and/or advanced glycation/lipoxidation end products (AGEs) in the peritoneal cavity and peritoneal tissue after intraperitoneal administration of a glucose-containing peritoneal dialysis fluid.

21. The use according to claim 20, wherein the carbonyl compound is 3-deoxyglucosone.

22. The use according to claim 20 or 21, wherein the advanced glycation/lipoxidation end product (AGE) is pentosidine.

23. The use according to any one of claims 20 to 22, wherein the oral medicinal composition is for improving a state of carbonyl stress in the peritoneal cavity and peritoneal tissue.

24. The use according to any one of claims 20 to 22, wherein the oral medicinal composition is for protecting the peritoneum of a peritoneal dialysis patient.

25. The use according to any one of claims 20 to 24, wherein the oral medicinal composition is for suppressing or/and improving peritoneal dysfunction or ultrafiltration failure in peritoneal dialysis patients.
